# EUROPEAN PATENT APPLICATION

(11) **EP 1 731 618 A1**
(43) Date of publication of application: **13.12.2006**
(21) Application number: 05425408.1
(22) Date of filing: 07.06.2005
(51) Int. Cl.: C12P 33/00

(54) **Process for the selective oxydation of colic acid**

(71) Applicant: PRODOTTI CHIMICI E ALIMENTARI SPA, 15060 Basaluzzo (Alessandria) (IT)
(72) Inventor: Fossati, Elena, Ist. di Chimica del Riconoscimento, 20131 Milano (IT); Carrea, Giacomo, Istituto di Chimica del, 20131 Milano (IT); Riva, Sergio, Ist. di Chimica del Riconoscimento, 20131 Milano (IT); Polentini, Fausto, 15060 Basaluzzo Alessandria (IT)
(74) Representative: Pistolesi, Roberto

(57) **Abstract**

There is described a new process for the enzymatic synthesis of 12-ketokenodeoxycholic acid by means of NAD-dependent enzymes. According to this new system, the oxidation reaction is brought about by means of the addition of NAD-dependent 12α-hydroxysteroid dehydrogenase (HSDH) to an aqueous solution which contains a water-soluble salt of cholic acid, together with catalytic quantities of NAD⁺, whose regeneration from NADH to NAD⁺ is brought about by means of the conversion of pyruvic acid into lactic acid using an NAD-dependent lactate dehydrogenase.

## Description

12-ketokenodeoxycholic acid which is obtained by chemical means from cholic acid which is extracted from bovine bile is used in the synthesis of kenodeoxycholic acid, which is in turn used as an intermediate in the synthesis of ursodeoxycholic acid. Ursodeoxycholic acid is known for its therapeutic properties and is used in the treatment of dysfunctions of the hepatic district.

Processes for converting 12-ketokenodeoxycholic acid into kenodeoxycholic acid are described, for example, in Fieser and Rajagopalan. JACS, 1950, 72, 5530-36 and in Alan F. Hofmann, Acta Chem. Scand. 17 (1963), 173-186, incorporated herein by reference; processes for converting kenodeoxycholic acid into ursodeoxycholic acid are instead described, for example, in the following publications, which are also incorporated herein by reference: B. Samuelsson, Acta Chem. Scand., 14 (1960) n°1 , 17-20; K. Kitahara, T. Sato (Tokyo Tanabe Co. Ltd) JP27,248/64; S. Kazuo, S. Shoichi, S. Koichiro (Tokyo Tanabe Co. Ltd) JP78,862/77; J. Bulidon, R. Demuynck, C. Pavan (Roussel-Uclaf) EP0072293A2; H. Masahiko, M. Kazutoshi, S. Tomio (Tokyo Tanabe Co. Ltd) JP32692/93.

12-ketokenodeoxycholic acid may be synthesized both by chemical means and by enzymatic means. The chemical process provides for esterification of cholic acid, selective acetylation of the hydroxyls at C-3 and C-7 and oxidation of the hydroxyl at C-12 to ketone; however, the yield and the conversion which can be obtained by chemical means are unsatisfactory from an industrial point of view. The oxidation of cholic acid by the use of enzymes, which is carried out as an alternative to chemical synthesis, instead allows 12-ketokenodeoxycholic acid to be obtained at quantitative yields and with a conversion rate of 100%. The process, which makes use of an NADP-dependent enzyme, such as glutamate dehydrogenase, is described by: Carrea G., Bovara R., Creminesi P., Lodi R. Biotechnology and Bioengineering 1984, 26, 560-563; Carrea G., Bovara R., Longhi R. and Riva S. Enzyme Microb. Technol. 1985, 7, 597-600; Riva S., Bovara R., Pasta P. and Carrea G. J. Org. Chem., 1986, 51, 2902-2906; Bovara R., Carrea G., Riva S. and Secundo F. Biotechnology Letters 1996, 18, 305-308; Carrea G., Ottolina G., Pasta P. and Riva S. Annals of the New YorkAcademy of Sciences. 1996, 799, 642-648.

However, the enzymatic methods set out above are also not entirely satisfactory owing to the high cost of the system for recycling the coenzyme, which is based on the use of α-ketoglutaric acid.

There has now been found a new process for the enzymatic synthesis of 12-ketokenodeoxycholic acid which, by using NAD-dependent enzymes, recycles the coenzyme, making use of a system which is substantially more economic than the previous one.

According to this new system, the oxidation reaction is brought about by means of the addition of NAD-dependent 12α-hydroxysteroid dehydrogenase (HSDH) to an aqueous solution which contains a water-soluble salt of cholic acid, together with catalytic quantities of NAD⁺, which is reduced to NADH, whose regeneration from NADH to NAD⁺ is brought about by means of the conversion of pyruvate into lactate using an NAD-dependent lactate dehydrogenase (LDH), as set out in scheme 1 below.

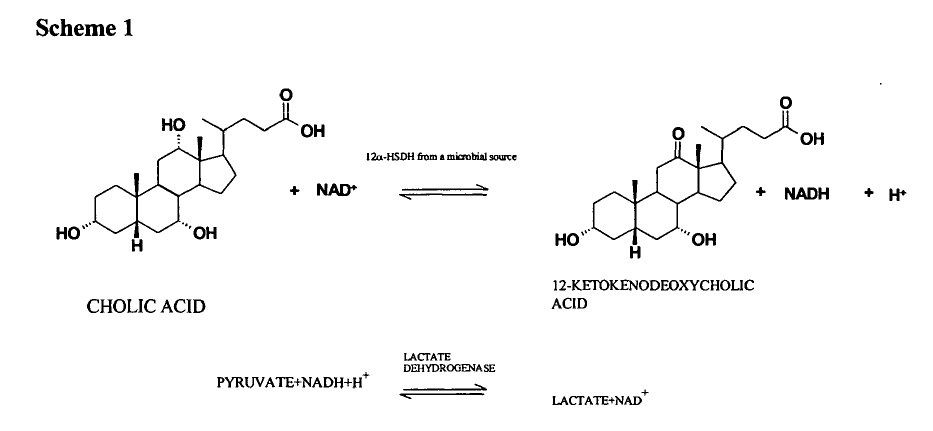

The process according to the present invention is carried out for a period of time of from 10 to 40 hours, by way of indication, and preferably from 18 to 36 hours, even more preferably from 20 to 24 hours.

The water-soluble salt of cholic acid is preferably the sodium salt; however, there can also be used other water-soluble salts, such as, for example, the potassium salt, lithium salt and ammonium salt.

The water-soluble salt of cholic acid is normally used at a concentration of from 1% to 6% by weight/volume; there are preferably used concentrations of from 2% to 5% and even more preferably from 3% to 4%.

The pH of the aqueous solution is preferably from 6 to 9, even more preferably from 7.5 to 8.5. That pH can be obtained by means of the buffers commonly used in the field, such as, for example, potassium phosphate, sodium bicarbonate, TRIS-HCl (tris(hydroxymethyl)aminomethane hydrochloric acid), TAPS (N-tris(hydroxymethyl) methyl-3-aminopropane sulphonic acid), ammonium formate.

The reaction temperature is preferably from 10°C to 40°C, preferably from 18°C to 35°C.

NAD⁺ is used in catalytic quantities, preferably at a concentration of from 1 to 1000µM, preferably from 10 to 100 µM.

Pyruvate is preferably used in the form of the sodium salt; it is normally used at least at equimolar quantities with respect to the salt of cholic acid, preferably in excess (estimable from 10 to 200%, preferably from 50 to 100%).

NAD-dependent 12α-HSDH may be that from *Eubacterium lentum,* which is described, for example, in Applied and Environmental Microbiology, May 1979, Vol. 37, no. 5, pages 992-1000 and in Biochimica et Biophysica Acta, 489, 1977, pages 466-476, incorporated herein by reference.

The NAD-dependent LDH preferably used is that from rabbit muscle; but there may be used other NAD-dependent lactate dehydrogenases, such as, for example, those from bovine muscle or bovine heart, from chicken heart, from porcine heart, from human erythrocytes, from *Leuconostoc mesenteroides,* from *Staphylococcus epidermidis,* from *Lactobacillus leichmanii,* from *Bacillus stearotermophilus;* those reagents are generally commercially available, for example, from Sigma-Aldrich.

12α-HSDH is normally used at quantities of from 1 to 100 U/ml, preferably from 10 to 30 U/ml; LDH is normally used at quantities of from 1 to 200 U/ml, preferably from 10 to 30 U/ml.

Once the reaction is complete, within times which preferably do not exceed 24 hours, the enzymes are separated by means of ultrafiltration and are recycled in a subsequent operation, and the 12-ketokenodeoxycholic acid is recovered by acidification and subsequent centrifuging.

The examples which follow must be considered to be non-limiting illustrations of the invention.

### Example 1

The reaction was carried out at ambient temperature and a pH of 8 with a total volume of 500 µl using: 4% by weight/volume of the sodium salt of cholic acid (0.1M, obtained by diluting an aqueous solution of the sodium salt of 0.5 M cholic acid at pH 8), 0.2 M of sodium pyruvate (100 µl from an aqueous solution of 1 M sodium pyruvate at pH 8), 16 units (U) of NAD-dependent 12α-hydroxysteroid dehydrogenase enzyme from a microbial source (40 µl from a 2 mg solution of lyophilized 12α-HSDH enzyme/400 µl of 0.1 M potassium phosphate buffer, pH 8 (40 U/ mg lyophilized)), 30 U of NAD-dependent lactate dehydrogenase enzyme from rabbit muscle (40 µl from a solution of 2 mg lyophilized lactate dehydrogenase enzyme/400 µl of 0.1 M potassium phosphate buffer, pH 8 (170 U/mg lyophilized)), 100 µM NAD⁺ (5 µl from a 10 mM NAD⁺ aqueous solution), 0.1 M potassium phosphate buffer, pH 8 (215 µl of 0.2 M potassium phosphate buffer, pH 8).

Complete conversion of cholic acid into 12-ketokenodeoxycholic acid is obtained within a reaction time of 24 hours. The degree of conversion was evaluated by means of TLC analyses using as the eluent system chloroform/methanol/acetic acid (10:1:0.5).

### Preparation of 0.5 M solution of sodium salt of cholic acid, pH 8

2.4 g of cholic acid were dissolved in a final volume of 12 ml of twice-distilled water by adding sodium hydroxide at 10% by weight/volume until complete dissolution of the salt. The pH of the solution was then brought to 8 by addition of 0.1 M HCl.

### Preparation of 0.5 M solution of sodium pyruvate, pH 8.

1.1 g of sodium pyruvate were dissolved in a final volume of 10 ml of twice-distilled water. The pH of the solution was then brought to 8 by addition of 0.1 M NaOH.

### Preparation of a 10 mM NAD⁺ solution

13.3 mg of NAD⁺ were dissolved in 2 ml of twice-distilled water.

### Example 2

Example 1 was repeated with various starting concentrations of cholic acid: 4 %, 4.5 %, 5 %, 5.5 % or 6 % by weight/volume.

Within 20 hours, there was obtained complete conversion of the substrate into a single product in the reaction containing 4% by weight/volume of cholic acid. Within 40 hours, there was also obtained complete conversion in the reaction containing 4.5% by weight/volume of cholic acid. In the reactions containing a concentration greater than 4.5% of cholic acid, however, there was observed no significant increase in the degree of conversion in the control carried out at 40 hours. The optimum concentration of cholic acid was therefore found to be the one containing 4% by weight/volume.

### Example 3

Example 1 was repeated at various pH values: pH 6.5, 7, 7.5, 8, 8.5 or 9.

Within 20 hours, there was obtained complete conversion of the substrate into the product in the reactions at a pH of 7.5, 8 and 8.5. The other reactions were found to be slower and in fact complete conversion was obtained within 40 hours for the reaction at pH 7 and within 5 days for the reactions at pH 6.5 and pH 9. The optimum pH range for the reaction was therefore found to be from 7.5 to 8.5.

### Example 4

Example 1 was repeated with various 0.1 M buffer solutions at pH 8: a buffer of potassium phosphate, sodium bicarbonate, TRIS-HCl (tris(hydroxymethyl)aminomethane hydrochloric acid), TAPS (N-tris(hydroxymethyl) methyl-3-aminopropane sulphonic acid), ammonium formate. Within 20 hours, irrespective of the buffer used, there was obtained complete conversion of the substrate into 12-ketokenodeoxycholic acid in all the reaction mixtures.

### Example 5

Example 1 was repeated at various reaction temperatures: ambient temperature (18° C), 25° C, 35° C or 45° C. In the reactions carried out at ambient temperature (18° C), at 25 °C and at 35°C, there was obtained complete conversion of the substrate into 12-ketokenodeoxycholic acid within 20 hours. In the reaction carried out at 45 °C, however, there was obtained a maximum conversion of approximately 50 %. From the control of the enzyme activity in the reaction mixtures, it was found that after 24 hours the activity of the lactate dehydrogenase enzyme at 45° C was 1/10 of the activity of the same enzyme measured during the reaction carried out at ambient temperature, and the activities of the 12α-HSDH enzyme in the reactions mentioned above were comparable. The lesser degree of conversion at 45 °C was therefore attributed to a reduction in the activity of the LDH enzyme at that temperature. The optimum reaction temperature must not therefore be greater than 35 °C.

### Example 6

Example 1 was repeated using various (NAD⁺) co-factor concentrations: 10 µM, 50 µM, 100 µM. Within 20 hours, there was obtained complete conversion of the substrate into the product in all the reaction mixtures; verification was carried out by means of TLC analyses (elution solvent: chloroform/methanol/acetic acid 10: 1: 0.5). Therefore, it is possible to obtain regioselective oxidation of cholic acid to form 12-ketokenodeoxycholic acid with a number of recycling operations of the NAD⁺ in the order of 10000.

### Example 7

Example 1 was repeated using various quantities of the two enzymes: 5 U of 12α-HSDH and 10 U of LDH; 10 U of 12α-HSDH and 20 U of LDH; 15 U of 12α-HSDH and 30 U of LDH; 15 U of 12α-HSDH and 15 U of LDH. Within 20 hours, there was obtained complete conversion of the substrate into the product in all the reaction mixtures. The reaction carried out in the presence of 5 U of 12α-HSDH and 10 U of LDH was found to be the slowest: after a reaction time of 14 hours, there was in fact still visible in TLC a small quantity of substrate, but which was completely used up in the other reaction mixtures (elution solvent: chloroform/methanol/acetic acid 10: 1: 0.5). In order to obtain complete conversion within 20 hours, however, it is possible to decrease to a quantity of 5 U of 12α-HSDH and 10 U of LDH.

## Claims

1. A process for the enzymatic synthesis of 12-ketokenodeoxycholic acid, wherein a water-soluble salt of cholic acid is caused to react with NAD-dependent 12α-HSDH in an aqueous solution and in the presence of catalytic quantities of NAD⁺, **characterized in that** the regeneration from NADH to NAD⁺ is brought about by means of the conversion of pyruvate into lactate using an NAD-dependent LDH.

2. A process according to claim 1, **characterized in that** the water-soluble salt of cholic acid is selected from the sodium salt, potassium salt, lithium salt and ammonium salt, preferably sodium salt.

3. A process according to either of the preceding claims, **characterized in that** the water-soluble salt of cholic acid has a concentration of from 1 to 6% by weight/volume, preferably from 2 to 5%, even more preferably from 3 to 4%.

4. A process according to any one of the preceding claims, **characterized in that** the NAD⁺ has a concentration of from 1 to 1000 µM, preferably from 10 to 100 µM.

5. A process according to any one of the preceding claims, **characterized in that** the pyruvate is sodium pyruvate.

6. A process according to any one of the preceding claims, **characterized in that** the pyruvate is at least at equimolar quantities with respect to the salt of cholic acid, preferably in excess.

7. A process according to any one of the preceding claims, **characterized in that** the NAD-dependent 12α-HSDH has a concentration of from 1 to 100 U/ml, preferably from 10 to 30 U/ml.

8. A process according to any one of the preceding claims, **characterized in that** the NAD-dependent 12α-HSDH is from *Eubacterium lentum.*

9. A process according to any one of the preceding claims, **characterized in that** the NAD-dependent LDH has a concentration of from 1 to 200 U/ml, preferably from 10 to 30 U/ml.

10. A process according to any one of the preceding claims, **characterized in that** the NAD-dependent LDH is from rabbit muscle, bovine muscle or bovine heart, from chicken heart, from porcine heart, from human erythrocytes, from *Leuconostoc mesenteroides,* from *Staphylococcus epidermidis,* from *Lactobacillus leichmanii,* from *Bacillus stearotermophilus,* preferably from rabbit muscle.

11. A process according to any one of the preceding claims, **characterized in that** the pH of the aqueous solution is from 6 to 9, preferably from 7.5 to 8.5.

12. A process according to any one of the preceding claims, **characterized in that** it is carried out for a period of time of from 10 to 40 hours, preferably from 18 to 36 hours, even more preferably from 20 to 24 hours.

13. A process according to any one of the preceding claims, **characterized in that** it is carried out at from 10 to 40° C, preferably from 18 to 35 °C.

14. A process for the preparation of ursodeoxycholic acid, **characterized in that** it comprises a process for the synthesis of 12-ketokenodeoxycholic acid according to any one of the preceding claims.
